Europäisches Patentamt

European Patent Office

Office européen des brevets

(19)

(11) **EP 1 096 865 B1**

(12) **EUROPÄISCHE PATENTSCHRIFT**

(45) Veröffentlichungstag und Bekanntmachung des Hinweises auf die Patenterteilung:
**26.10.2005 Patentblatt 2005/43**

(21) Anmeldenummer: **99934673.7**

(22) Anmeldetag: **13.07.1999**

(51) Int Cl.[7]: **A23L 3/3463**

(86) Internationale Anmeldenummer:
**PCT/EP1999/004896**

(87) Internationale Veröffentlichungsnummer:
**WO 2000/003612 (27.01.2000 Gazette 2000/04)**

(54) **ANTIMIKROBIELLE ZUSAMMENSETZUNG**

ANTIMICROBIAL COMPOSITION

COMPOSITION ANTIMICROBIENNE

(84) Benannte Vertragsstaaten:
**AT BE CH CY DE DK ES FI FR GB GR IE IT LI LU MC NL PT SE**

(30) Priorität: **13.07.1998 DE 19831288**
       **13.07.1998 DE 19831306**
       **13.07.1998 DE 19831309**

(43) Veröffentlichungstag der Anmeldung:
**09.05.2001 Patentblatt 2001/19**

(73) Patentinhaber: **Schuer, Joerg Peter**
**41844 Wegberg-Dalheim (DE)**

(72) Erfinder: **Schuer, Joerg Peter**
**41844 Wegberg-Dalheim (DE)**

(74) Vertreter: **Helbing, Jörg et al**
**Patentanwälte**
**von Kreisler-Selting-Werner,**
**Postfach 10 22 41**
**50462 Köln (DE)**

(56) Entgegenhaltungen:
**WO-A-96/29895          US-A- 4 927 651**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

Printed by Jouve, 75001 PARIS (FR)

**Beschreibung**

Die vorliegende Erfindung betrifft eine antimikrobielle Zusammensetzung, deren Verwendung zur Haltbarkeitsverbesserung und/oder zur Stabilisierung von mikrobiell verderblichen Produkten, deren Verwendung als Prozesshilfsmittel sowie mikrobiell verderbliche Produkte, die die antimikrobielle Zusammensetzung enthalten.

Industriell bearbeitete Nahrungs- und Futtermittel, Kosmetika, Pharmazeutika und andere für mikrobielle Verderbnis anfällige Produkte müssen eine gewisse, nicht zu kurze Zeit haltbar sein, um nach einem Transport und Vertrieb über die üblichen Wege unverdorben den Verbraucher zu erreichen. Der Verbraucher erwartet darüber hinaus, dass das erworbene Produkt auch nach dem Kauf nicht sofort verdirbt, sonderm, je nach Produkt, einige Tage oder Wochen auf Vorrat gehalten werden kann.

Unbehandelt würden die meisten Nahrungs- und Futtermittel innerhalb weniger Tage verderben, da sich Pilze und/oder Bakterien ungehindert, allenfalls durch Kühlung beeinträchtigt, auf einem für sie idealen Nährboden vermehren könnten. Typische Beispiele sind der Verderb von Brot durch Schimmelpilze, z.B. Aspergillus niger, von Fleischprodukten (z.B. Wurst) durch Enterobakterien oder Lactobacillen, die Kontamination von Geflügel durch Salmonellen und vieles andere mehr. Da Pilze einschließlich Hefen bzw. deren Sporen, Grampositive und Gramnegative Bakterien überall vorhanden sind, wo nicht durch besondere, kostspielige und industriell aus ökonomischen Erwägungen nicht anwendbare Maßnahmen ein steriles Umfeld geschaffen wird, müssen geeignete Gegenmaßnahmen getroffen werden.

Herkömmlicherweise werden daher Nahrungs- und Futtermittel, Kosmetika, Pharmazeutika, Farben, Papier und Zellstoffe und andere verderbliche Produkte mit Konservierungsmitteln haltbar gemacht, die laut der Codex Alimentarius Liste der Food und Agriculture Organisation (FAO/WHO Food Standard Programme) in Division 3 Food Additives Preservatives 3.37 als "synthetische Konservierungsmittel" aufgeführt und meist in Form von chemischen Monosubstanzen oder deren Kombinationen eingesetzt werden.

Aus dem Stand der Technik ist eine Vielzahl von Additiven zur Konservierung von verderblichen Produkten bekannt. Hierzu zählen z.B. Additive auf der Basis von Aromastoffen, Alkoholen, organischen Säuren, Aldehyden, phenolischen Stoffen und ätherischen Ölen. Solche Zusammensetzungen sind beispielsweise in der US-Patentschrift 4,446,161, US-4,927,651, WO-94/14414, derGB-172,993 und der DE-OS-31 38 277 sowie in E. Lück (Chemische Lebensmittelkonservierung, Seite 1977, 1986 Springer-Verlag) beschrieben.

Die in der erwähnten Liste aufgeführten Konservierungsmittel sind bakteriostatisch und/oder fungistatisch wirksam und verbessern die Haltbarkeit wesentlich. Sie werden jedoch von vielen Verbrauchern abgelehnt, da ihre Auswirkungen auf die Gesundheit des Verbrauchers nicht bekannt sind, bzw. schädliche Einflüsse, insbesondere bei wiederholter Aufnahme über einen langen Zeitraum, nicht ausgeschlossen werden können. Nachteilig ist auch, daß alle bisher bekannten Verfahren auf der Änderungen des pH-Werts oder $a_w$-Werts beruhen.

Als Lösung dieses Problems schlägt die WO-96/29895 antimikrobielle Zusammensetzungen mit mehreren GRAS (Generally Recognized As Safe)-Aromastoffen vor. In diesen Zusammensetzungen werden einerseits nur die lebensmittelrechtlich, unbedenklichen GRAS-Aromastoffe verwendet. Darüber hinaus konnte ein synergistischer antimikrobieller Effekt beobachtet werden, aufgrund dessen weitaus geringere Mengen der Aromastoffe (Konservierungsmittel) eingesetzt werden können.

In der WO-98/58590 (veröffentlicht 30. Dezember 1998) werden weitere antimikrobielle Zusammensetzungen beschrieben, in denen Gemische aus Polyphenol und einem GRAS-Aroma-Alkohol bzw. aus Benzylalkohol und einem weiteren GRAS-Aroma-Alkohol noch weitere Komponenten wie (a) einwertige oder mehrwertige Alkohole mit 2 bis 10 C-Atomen, (b) organische Säuren mit 1 bis 15 C-Atomen oder deren physiologischen Salze und/oder (c) wasserlösliche Lösungsvermittler, insbesondere Glycerin oder Propylenglycol, zugefügt sind.

Nachteilig bei diesen Konservierungsmitteln ist insbesondere, dass sie regelmäßig in hohen Konzentrationen dem Nahrungsmittel zugegeben werden. Dadurch gelangen relativ große Mengen dieser Mittel beim Verzehr auch in den menschlichen Körper. Die Folge sind die heute vielfach gehäuft auftretenden Reaktionen in Form allergischer Erkrankungen.

Eine Alternative zur Konservierung durch Zusatz von synthetischen Konservierungsmitteln ist die thermische Inaktivierung von Keimen, z.B. durch Pasteurisieren. Unter Pasteurisieren versteht man eine thermische Behandlung von 30 bis 120 Minuten Einwirkzeit bei 70 bis 85°C.

Die Pasteurisierung verbessert die Haltbarkeit derart behandelter Produkte erheblich, ist jedoch technisch aufwendig und verbraucht sehr viel Energie. Die Lebensfähigkeit von Sporen wird darüber hinaus oft nicht oder nur sehr unvollständig beeinträchtigt. Eine Pasteurisierung ist außerdem für temperaturempfindliche Produkte nicht anwendbar oder führt zu einem nicht unerheblichen Qualitätsverlust, da spätestens durch das oftmals notwendige zweite Thermisieren (bis 85°C) der "Frischegrad" des pasteurisierten Produktes nachläßt. Außerdem sind gerade wertvolle Bestandteile von Nahrungsmitteln, Kosmetika oder Pharmazeutika, z.B. Vitamine, Aminosäuren und viele pharmazeutische Wirkstoffe, thermolabil, so dass sich eine thermische Behandlung unter den üblichen Pasteurisierungsbedingungen verbietet.

Eine weitere Möglichkeit zur Verbesserung der Haltbarkeit ist es, das von Verderbnis bedrohte Produkt unter Stickstoff oder $CO_2$ luftdicht zu verpacken oder in Vakuumverpackungen bereitzustellen, wie es z.B. bei gemahlenem Kaffee gehandhabt wird. Diese Verfahren sind jedoch teuer und aufwendig und daher für viele Nahrungsmittel nicht anwendbar.

Aufgabe der vorliegenden Erfindung ist es demgemäß, ein Additiv zur Haltbarkeitsverbesserung und/oder zur Stabilisierung von mikrobiell verderblichen Produkten zur Verfügung zu stellen, das die genannten Nachteile des Standes der Technik nicht aufweist.

Überraschenderweise wurde gefunden, dass der antimikrobielle Effekt der in der WO 96/29895 beschriebene Zusammensetzung aus GRAS-Aromastoffen weiter gesteigert werden kann, wenn einer der Bestandteile der Zusammensetzungen ein hydrophiler GRAS-Aromastoff ist.

Gegenstand der vorliegenden Anmeldung ist demgemäß

(1) eine antimikrobielle Zusammensetzung, die

(A) ein Gemisch ist, das wenigstens zwei GRAS (Generally Recognized As Safe)-Aromastoffe, ausgenommen Polyphenolverbindungen und Benzylalkohol und wenigstens einen hydrophilen, nicht-alkoholischen GRAS-Aromastoff umfasst; oder

(B) ein Gemisch ist, das Benzylalkohol oder Polyphenolverbindungen und wenigstens einen nicht-alkoholischen, hydrophilen GRAS-Aromastoff umfasst, wobei das Gemisch keine weiteren GRAS-Aroma-Alkohole enthält,

wobei der hydrophile, nicht-alkoholische GRAS-Aromastoff eine organische Säure mit 1 bis 15 C-Atomen oder ein physiologisches Salz derselben, ein hydrophiles Acetat oder ein hydrophiler Aldehyd ist und wobei das Gemisch (A) mindestens zwei lipophile GRAS-Aroma-Alkohole, ausgenommen Benzylalkohol umfasst;

(2) ein Verfahren zur Haltbarkeitsverbesserung und/oder Stabilisierung von mikrobiell verderblichen Produkten, dadurch gekennzeichnet, dass eine antimikrobielle Zusammensetzung, wie vorstehend in (1) definiert, dem mikrobiell verderblichen Produkt als Additiv zugesetzt wird;

(3) die Verwendung der antimikrobiellen Zusammensetzung, wie vorstehend in (1) definiert, als Additiv für mikrobiell verderbliche Produkte, insbesondere als Additiv für Lebensmittel und Kosmetika;

(4) ein Verfahren zur Haltbarkeitsverbesserung und/oder Stabilisierung von mikrobiell verderblichen Produkten, bei dem vor, nach oder während des Prozesses zur Herstellung, Verarbeitung oder Verpackung der Produkte deren Oberflächen und/oder deren Umgebung, insbesondere die Umgebungsluft und/oder die Oberflächen der unmittelbar mit den Produkten in Kontakt kommenden Geräte oder sonstigen Materialien mit einem oder mehreren Prozesshilfsmitteln beaufschlagt werden, dadurch gekennzeichnet, dass das Prozesshilfsmittel eine, wie vorstehend in (1) definierte, antimikrobielle Zusammensetzung umfaßt;

(5) die Verwendung der antimikrobiellen Zusammensetzung, wie vorstehend in (1) definiert, als Prozesshilfsmittel; und

(6) ein mikrobiell verderbliches Produkt, insbesondere in Lebensmitteln, Kosmetika oder Pharmazeutika, enthaltend die vorstehend in (1) definierte antimikrobielle Zusammensetzung.

Im folgenden werden die erfindungsgemäßen Zusammensetzungen (A) und (B) im einzelnen näher beschrieben:

Die GRAS-Aromastoffe der Gemische (A) und (B) sind von der FDA-Behörde zur Verwendung in Nahrungsmitteln als gewerbesicher anerkannt (GRAS = Generally Recognized As Safe In Food). Bei den erwähnten GRAS-Aroma-Alkoholen und GRAS-Aromastoffen handelt es sich um solche Verbindungen, die in FEMA/FDA GRAS Flavour Substances Lists GRAS 3-15 Nr. 2001-3815 (Stand 1997) genannt sind. In dieser Liste sind natürliche und naturidentische Aromastoffe aufgeführt, die von der amerikanischen Gesundheitsbehörde FDA zur Verwendung in Nahrungsmitteln zugelassen sind: FDA Regulation 21 CFR 172.515 für naturidentische Aromastoffe (Synthetic Flavoring Substances and Adjuvants) und FDA Regulation 21 CFR 182.20 für natürliche Aromastoffe (Natural Flavoring Substances and Adjuvants).

Das Gemisch (A) kann weiterhin einen hydrophilen, alkoholischen GRAS-Aromastoff enthalten. Hierbei handelt es sich um einwertige oder mehrwertige Alkohole mit 2 bis 10, vorzugsweise mit 2 bis 7 C-Atomen. Besonders bevorzugt sind 1-Propanol (Propylalkohol), Glycerin, Propylenglycol, Acetoin, (Acetylmethylcarbinol), Ethanol und 2-Propanol (Isopropanol). Bei Verwendung der antimikrobiellen Zusammensetzung zur Behandlung von Lebensmitteln oder als Additiv in Lebensmitteln, ist es jedoch empfehlenswert, den Ethanol- bzw. 2-Propanolgehalt möglichst gering zu halten, bzw. vollständig darauf zu verzichten.

Die organische Säure weist vorzugsweise 2 bis 10 C-Atome auf. Besonders bevorzugt sind Essigsäure, Aconitsäure, Ameisensäure, Apfelsäure, (Hydroxybernsteinsäure), Milchsäure, Phenylessigsäure (α-Toluolsäure), Citronensäure, Mandelsäure (Hydroxyphenylessigsäure), Weinsäure, Fumarsäure, Tanninsäure, Hydrozimtsäure (3-Phenyl-1-propionsäure) und deren physiologischen Salze. Die physiologischen Salze umfassen dabei die Alkali-, Erdalkali- und Ammoniumsalze.

Bei den hydrophilen GRAS-Acetaten handelt es sich vorzugsweise um Allicin, Triacetin (Glycerintriacetat), Kaliumacetat, Natriumacetat und Calciumacetat. Die hydrophilen GRAS-Aldehyde sind vorzugsweise Furfurol, Propionaldehyd (Propanal) und Vanillin.

Die GRAS-Aromastoffe des Gemisches (A) sind vorzugsweise lipophile GRAS-Aromastoffe. Insbesondere sind die GRAS-Aromastoffe des Gemisches (A) ausgewählt aus den folgenden Bestandteilen (a) lipophilen Alkoholen, (b) Phenolen, (c) Estern, (d) Terpenen, (e) Acetalen, (f) lipophilen Aldehyden, (g) etherischen Ölen, (h) lipophilen Säuren und deren Derivate.

Erfindungsgemäß kann das Gemisch (A) mindestens zwei lipophile GRAS-Aroma-Alkohole (a) oder deren Derivate enthalten. Bevorzugt wird erfindungsgemäß der Einsatz von zwei oder drei GRAS-Aroma-Alkoholen. Im einzelnen können beispielsweise folgende lipophile GRAS-Aroma-Alkohole zum Einsatz kommen:

n-Butylalkohol (n-Propylcarbinol), iso-Butylalkohol (2-Methyl-1-propanol), Hexylalkohol (Hexanol), L-Menthol, Octylalkohol (n-Octanol), Zimtalkohol (3-Phenyl-2-propen-1-ol), $\alpha$-Methylbenzylalkohol (1-Phenylethanol), Heptylalkohol (Heptanol), n-Amylalkohol (1-Pentanol), iso-Amylalkohol (3-Methyl-1-butanol), Anisalkohol (4-Methoxybenzylalkohol, p-Anisalkohol), Citronellol, n-Decylalkohol (n-Decanol), Geraniol, $\beta$-$\gamma$-Hexenol (3-Hexenol), Laurylalkohol (Dodecanol), Linalool, Nerolidol, Nonadienol (2,6-Nonadien-1-ol), Nonylalkohol (Nonanol-1), Rhodinol, Terpineol, Borneol, Clineol (Eucalyptol), Anisol, Cuminylalkohol (Cuminol), 10-Undecen-1-ol, 1-Hexadecanol.

Als Derivate können sowohl natürliche oder naturidentische Derivate als auch synthetische Derivate eingesetzt werden. Geeignete Derivate sind z. B. die Ester, Ether und Carbonate der vorstehend genannten GRAS-Aroma-Alkohole. Falls in dem Gemisch (A) die wenigstens zwei GRAS-Aromastoffe ausschließlich GRAS-Aroma-Alkohole sind, wird jedoch vorzugsweise ein hydrophiler, nicht-alkoholischer GRAS-Aromastoff verwendet.

Als Bestandteile (b) können folgende Phenolverbindungen zum Einsatz kommen:

Thymol, Methyleugenol, Acetyleugenol, Safrol, Eugenol, Isoeugenol, Anethol, Phenol, Methylchavicol (Estragol; 3-4-Methoxyphenyl-1-propen), Carvacrol, $\alpha$-Bisabolol, Fornesol, Anisol (Methoxybenzol) und Propenylguaethol (5-Prophenyl-2-ethoxaphenol) und deren Derivate. Derivate der Phenolverbindungen im Sinne der vorliegenden Erfindung sind Verbindungen, in denen die phenolische Hydroxylgruppe verestert oder verethert ist.

Als lipophile Ester (Bestandteil (c)) kommen die folgenden Acetate zum Einsatz: Iso-Amylacetat (3-Methyl-1-butylacetat), Benzylacetat, Benzylphenylacetat, n-Butylacetat, Cinnamylacetat (3-Phenylpropenylacetat), Citronellylacetat, Ethylacetat (Essigester), Eugenolacetat (Acetyleugenol), Geranylacetat, Hexylacetat (Hexanylethanoat), Hydrocinnamylacetat (3-Phenyl-propylacetat), Linalylacetat, Octylacetat, Phenylethylacetat und Terpinylacetat. Weitere geeignete Ester sind Esterderivate der vorstehend definierten hydrophilen GRAS-Aromasäuren und der lipophilen GRAS-Aromasäure (Bestandteil (h)), z.B. deren $C_{1-6}$-Alkylester und Benzylester.

Als Terpene (Bestandteil (d)) kommen z. B. Campher, Limonen und $\beta$-Caryophyllen in Betracht.

Zu den verwendbaren Acetalen (Bestandteil (e)) zählen z. B. Acetal, Acetaldehyddibutylacetal, Acetaldehyddipropylacetal, Acetaldehydphenethylpropylacetal, Zimtaldehydethylenglycolacetal, Decanaldimethylacetal, Heptanaldimethylacetal, Heptanalglycerylacetal und Benzaldehydpropylenglykolacetal.

Als lipophile Aldehyde (Bestandteil (f)) sind z. B. Acetylaldehyd, Anisaldehyd, Benzaldehyd, iso-Butylaldehyd (Methyl-1-propanal), Citral, Citronellal, n-Caprinaldehyd (n-Decanal), Ethylvanillin, Heliotropin (Piperonal), Heptylaldehyd (Heptanal), Hexylaldehyd (Hexanal), 2-Hexenal ($\beta$-Propylacrolein), Hydrozimtaldehyd (3-Phenyl-1-propanal), Laurylaldehyd (Dodecanal), Nonylaldehyd (n-Nonanal), Octylaldehyd (n-Octanal), Phenylacetaldehyd (1-Oxo-2-phenylethan), Zimtaldehyd (3-Phenylpropenal), Perillaaldehyd und Cuminaldehyd verwendbar.

Erfindungsgemäß einsetzbar sind auch die im folgenden aufgeführten etherischen Öle und/oder die alkoholischen, glykolischen oder durch $CO_2$-Hochdruckverfahren erhaltenen Extrakte aus den genannten Pflanzen (Bestandteil (g)):

(g1) Öle bzw. Extrakte mit hohem Anteil an Alkoholen: Melisse, Koriander, Kardamon, Eukalyptus;
(g2) Öle bzw. Extrakte mit hohem Anteil an Aldehyden: Eukalyptus citriodora, Zimt, Zitrone, Lemongras, Melisse, Citronella, Limette, Orange;
(g3) Öle bzw. Extrakte mit hohem Anteil an Phenolen: Oreganum, Thymian, Rosmarin, Orange, Nelke, Fenchel, Campher, Mandarine, Anis, Cascarille, Estragon und Piment;
(g4) Öle bzw. Extrakte mit hohem Anteil an Acetaten: Lavendel;
(g5) Öle bzw. Extrakte mit hohem Anteil an Estern: Senf, Zwiebel, Knoblauch;

(g6) Öle bzw. Extrakte mit hohem Anteil an Terpenen: Pfeffer, Pomeranze, Kümmel, Dill, Zitrone, Pfefferminz, Muskatnuß.

Als lipophile Säuren (Bestandteil (h)) können die folgenden Säuren verwendet werden: Adipinsäure, Capronsäure, Pelargonsäure (Nonansäure), Valeriansäure (Pentansäure), iso-Valeriansäure (3-Methylbutansäure), Phenoxyessigsäure (Glycolsäurephenylether), Zimtsäure (3-Phenylpropensäure), deren Derivate, wie Amide (einschließlich N-substituierte Amide) und Salze (Alkali-, Erdalkali- und Ammoniumsalze) sowie lipophile Derivate der vorstehend erwähnten hydrophilen Säuren [z.B. deren N-substituierte Amide und an den Seitenketten-Hydroxyfunktionen modifizierte (acylierte und alkylierte) Verbindungen].

Das Gemisch (A) enthält vorzugsweise 0,01 bis 90 Gew.-%, vorzugsweise 0,1 bis 50 Gew.-% GRAS-Aromastoffe (a) bis (h). Besonders bevorzugt ist, wenn das Gemisch (A) 0,01 bis 30 Gew.-%, vorzugsweise 0,1 bis 10 Gew.-% lipophile GRAS-Aromastoffe (a) bis (h) enthält.

Der Anteil der hydrophilen, nicht-alkoholischen GRAS-Aromastoffe darf dabei bis zu 90 Gew.-% des Gemisches (A) betragen und beträgt vorzugsweise 0,1 bis 50 Gew.-%.

Dabei liegt das Mischungsverhältnis der einzelnen Bestandteile des Gemisches (A) [hydrophile, alkoholische GRAS-Aromastoffe; hydrophile, nicht-alkoholische GRAS-Aromastoffe; Bestandteile (a) bis (h)] zwischen 10.000:1 und 1:10.000, vorzugsweise zwischen 1000:1 und 1:1000 und besonders bevorzugt zwischen 100:1 und 1:100.

Besonders bevorzugt als Gemisch (A) ist eine Zusammensetzung, die mindestens zwei der vorstehend definierten GRAS-Aroma-Alkohole (Bestandteil (a)) und wenigstens einen der hydrophilen, nicht-alkoholischen GRAS-Aromastoffe enthält.

In dem Gemisch (B) werden vorzugsweise lipophile Polyphenolverbindungen, insbesondere die folgenden Polyphenole eingesetzt:

Brenzcatechin, Resorcin, Hydrochinon, Phloroglucin, Pyrogallol, Cyclohexan, Usninsäure, Acylpolyphenole, Lignine, Anthocyane, Flavone, Catechine, Gallussäurederivate (z. B. Tannine, Gallotannin, Gerbsäuren, Gallus-Gerbsäuren), deren Derivate wie (2,5-Dihydroxyphenyl)carboxyl- und (2,5-Dihydroxyphenyl)alkylencarboxylsubstitutionen, Salze, Ester und Amide, Kaffeesäure und deren Ester und Amide, Flavonoide (z. B. Flavon, Flavonol, Isoflavon, Gossypetin, Myrecetin, Robinetin, Apigenin, Morin, Taxifolin, Eriodictyol, Naringin, Rutin, Hesperidin, Troxerutin, Chrysin, Tangeritin, Luteolin, Catechine, Quercetin, Fisetin, Kaempferol, Galangin, Rotenoide, Aurone, Flavonole, Diole), Extrakte aus z. B. Camellia Primula. Weiterhin können auch deren mögliche Derivate, z. B. Salze, Säuren, Ester, Oxide und Ether verwendet werden. Das besonders bevorzugte Polyphenol ist Tannin (eine GRAS-Verbindung).

Das Gemisch (B) besteht vorzugsweise aus 0,01 bis 99 Gew.-%, vorzugsweise 0,1 bis 90 Gew.-% .Benzylalkohol oder Polyphenolverbindungen und 0.01 bis 50 Gew.-%, vorzugsweise 0,1 bis 30 Gew.-% hydrophilen, nicht-alkoholischen GRAS-Aromastoffen. Daneben kann das Gemisch (B) noch weitere, vorzugsweise lipophile GRAS-Aromastoffe, wie die vorstehend definierten (b) Phenole, (c) lipophile Ester, (d) Terpene, (e) Acetale, (f) lipophile Aldehyde, (g) etherische Öle und (h) lipophile Säuren und deren Derivate enthalten.

Bevorzugt werden als weitere GRAS-Aromastoffe Phenole (b) und/oder etherische Öle (g) verwendet.

Der Anteil der weiteren Bestandteile (b) bis (h) im Gemisch (B) darf bis zu 50 Gew.-% betragen, liegt jedoch vorzugsweise im Bereich von 0,001 bis 25 Gew.-% und besonders bevorzugt im Bereich von 0,01 bis 9 Gew.-%. Dabei liegt das Mischungsverhältnis der einzelnen Bestandteile des Gemisches (B) [Benzylalkohol oder Polyphenolverbindungen; nicht-alkoholische GRAS-Aromastoffe; Bestandteile (b) bis (h)] zwischen 10.000:1 und 1:10.000, vorzugsweise zwischen 1000:1 und 1:1000 und besonders bevorzugt zwischen 100:1 und 1:100.

Besonders bevorzugt im Sinne der vorliegenden Erfindung sind antimikrobielle Zusammensetzungen, deren antimikrobiell wirksamer Bestandteil ausschließlich aus GRAS-Aromastoffen besteht, d. h. keine "Derivate" der GRAS-Aromastoffe enthält. Dies ist besonders wichtig, wenn die antimikrobielle Zusammensetzung mit Nahrungsmitteln in Kontakt kommt.

In einer anderen Ausführungsform kann die Zusammensetzung weiterhin Emulgatoren, Stabilisatoren, Antioxidantien, Konservierungsmittel, Lösemittel und/oder Trägerstoffe enthalten.

Die erhöhte antimikrobielle Aktivität der Gemische (A) und (B) beruht auf der Tatsache, dass die meisten Aromastoffe ausschließlich fettlöslich (lipophil) sind. Zur Verwendung speziell im Nahrungsmittelbereich ist es dennoch erforderlich, dass Synergismen mit hydrophilen Aromastoffen vorliegen, da die lipophilen Aromastoffe sonst ihre mikrobizide Wirksamkeit nur unzureichend in zumeist überwiegend wasserhaltigen Nahrungsmitteln und Rohstoffen entfalten können.

So ist es notwendig, dass mindestens ein Aromastoff zusätzlich hydrophil ist, um gegebenenfalls lipophile andere Aromastoffe als Lösungsvermittler in Synergismen aufzunehmen, um dann gemeinsam an, in oder auf Nahrungsmitteln und/oder Rohstoffen mikrobizid zu wirken.

Andererseits lösen sich hydrophile Verbindungen, wie GRAS-Aromasäuren alleine nur in ausschließlich wasserhaltigen Lebensmitteln. Da Nahrungsmittel und Rohstoffe auch zumeist Fett enthalten, ist es notwendig, dass eine Zusammensetzung aus Aromastoffen lipophile Eigenschaften aufweist.

Die antimikrobielle Aktivität der erfindungsgemäßen Zusammensetzung beruht auf dem folgendem neuen Wirkprinzip: Die Zusammensetzung erlaubt die Penetration der Bestandteile in dem Mikroorganismus, unterbindet damit dessen Vermehrung, zerstört ihn jedoch nicht, wie dies z.B. Konservierungsmittel oder Ethanol als Einzelsubstanz in Form von Koagulation (Zerstörung) des Eiweißes des Mikroorganismus tun.

Die erfindungsgemäßen antimikrobiellen Zusammensetzungen sind somit als Additive zur Haltbarkeitsverbesserung und/oder Stabilisierung von mikrobiell verderblichen Produkten, wie Nahrungs- und Futtermittel, Pharmazeutika und Kosmetika geeignet. Insbesondere sind sie als Additive für die folgenden Gruppen von Nahrungsmitteln geeignet:

Brot, Backwaren, Backmittel, Backpulver, Puddingpulver, Getränken, diätischen Lebensmitteln, Essenzen, Feinkost, Fisch und Fischprodukten, Kartoffeln und Produkten auf Kartoffelgrundlage, Gewürzen, Mehl, Margarine, Obst und Gemüse und Produkten auf Grundlage von Obst und Gemüse, Sauerkonserven, Stärkeprodukten, Süßwaren, Suppen, Teigwaren, Fleisch- und Fleischwaren, Milch-, Molkerei- und Käseprodukten, Geflügel und Geflügelprodukten, Ölen, Fetten und öl- oder fetthaltigen Produkten.

Die erfindungsgemäßen Additive sind insbesondere gegen Schimmelpilze, Hefen und Bakterien (Grampositive und Gramnegative) wirksam. Vor allem gegen pathogene Erreger (Enterobacteriaceaen, z.B. E. Coli, Salmonellen, Enterokokken Listerien, z.B. Staphylokokken, Streptokokken) wie auch gegen Verderbniserreger, wie z.B. Aspergillus niger, Hefen, z.B. Endomyces tibuliger, wirken sie hervorragend. Ebenso wirken die erfindungsgemäße Additive auf Viren und reduzierend gegen mikrobielle Toxine, z.B. Aflatoxine, Enterotoxine.

Die Additive werden vorzugsweise in Mengen von 1 ppm bis 10 Gew.-%, vorzugsweise 1 ppm bis 1,0 Gew.-% dem mikrobiell verderblichen Produkt zugesetzt. Besonders bevorzugte Mengen sind 0,001 Gew.-% bis 0,5 Gew.-%. Ganz besonders bevorzugt sind 0,002 Gew.-% bis 0,25 Gew.-%.

Es ist erfindungsgemäß überraschend, dass die Wirkung der erfindungsgemäßen Additive bereits bei Anwendung der genannten geringen Konzentrationen auftritt. Dies ist um so überraschender, als die mit den erfindungsgemäßen Additiven behandelten Nahrungsmittel eine erheblich längere Haltbarkeit aufweisen als die mit herkömmlichen Konservierungsstoffen behandelten verderblichen Produkte.

Überraschend ist auch, dass die beschriebenen Vorteile schon bei mikrobischen Einwirkzeiten von weniger als 24 h, insbesondere als 60 Minuten, vorzugsweise 1-60 Minuten, höchst bevorzugt 5-15 Minuten auftreten.

Die erfindungsgemäßen Additive führen überraschenderweise zu keinen Nachteilen im Geschmack, Geruch oder Farbe bei dem behandelten Nahrungsmittel. Ein besonderer Vorteil gegenüber dem bisherigen Stand der Technik ist, dass keinerlei Verschiebung des pH-Werts oder $a_w$-Werts zu verzeichnen ist. D.h.; die Wirkung der eingesetzten Additive ist überraschenderweise unabhängig von pH-Wert und $a_w$-Wert. Ebenso überraschend ist es, dass die Additive unabhängig von der Feuchtigkeit, dem Fett-, Eiweiß- und Kohlenhydratgehalt verwendbar sind. Schließlich sind die erfindungsgemäßen Kombinationen unempfindlich gegen Temperaturschwankungen im Bereich zwischen -30°C und 200°C, d.h. sowohl kälteals auch hitzeunempfindlich.

Die Additive können den mikrobiell verderblichen Produkten auch in Form von Retardierungsformulierungen zugegeben sein. Eine geeignete Retardzubereitung ist z.B. die Mikroverkapselung der antimikrobiellen Zusammensetzung. Als Mikroverkapselungsmaterial kann dabei Maltodextrose oder Cellulosederivat verwendet werden.

Daneben kann die erfindungsgemäße antimikrobielle Zusammensetzung auch als Prozesshilfsmittel für die Verarbeitung der vorstehend definierten mikrobiell verderblichen Produkte eingesetzt werden. Dies bedeutet, dass vor, während oder nach Abschluß des Prozesses zur Herstellung, Verarbeitung oder Verpackung der Produkte deren Oberflächen und/oder deren Umgebung, insbesondere die Umgebungsluft und/oder die Oberflächen der unmittelbar oder mittelbar mit den Produkten in Kontakt kommenden Geräte oder sonstige Materialien mit einem oder mehreren Prozesshilfsmitteln beaufschlagt werden.

"Beaufschlagen" im Sinne der vorliegenden Erfindung umfaßt die folgenden Bearbeitungsvorgänge: Aufstreichen, Schmieren, Emulgieren, Trennen, Reinigen, Sprühen, Vernebeln, Vergasen, Schneiden, Tauchen und Marinieren.

Die Anwendung der antimikrobiellen Zusammensetzung als Prozesshilfsmittel erfolgt unverdünnt, in wasserlöslicher Verdünnung mit Wasser, in lebensmittelzulässigen Lösemitteln (z.B. Alkoholen) oder in fettlöslichen Verdünnungen mit Pflanzenfetten oder Ölen.

Bevorzugt ist dabei eine Verwendung der Prozesshilfsmittel für die Produktion in Nahrungs- und Futtermitteln, Kosmetika, Pharmazeutika, Farben, Papier und/oder Zellstoffen.

In besonders bevorzugten Ausführungsformen werden die Prozesshilfsmittel zur Haltbarkeitsverbesserung und Stabilisierung von aus der folgenden Gruppe ausgewählten Nahrungsmitteln verwendet:

Brot, Backwaren, Backmitteln, Backpulver, Puddingpulver, Getränken, diätetischen Lebensmitteln, Essenzen, Feinkost, Fisch und Fischprodukten, Kartoffeln und Produkten auf Kartoffelgrundlage, Gewürzen, Mehl, Margarine, Obst und Gemüse und Produkten auf Grundlage von Obst und Gemüse, Sauerkonserven, Stärkeprodukten, Süßwaren, Suppen, Teigwaren, Fleisch- und Fleischwaren, Milch-, Molkerei- und Käseprodukten, Geflügel und Geflügelprodukten, Ölen, Fetten und öl- oder fetthaltigen Produkten.

Das Prozesshilfsmittel wirkt im Umfeld des für Verderbnis anfälligen Produktes, beispielsweise eines Nahrungs- oder Futtermittels, z.B. auf Maschinenteilen, die in Kontakt mit dem zu be- oder verarbeitenden Produkt stehen, oder in der Luft. Durch den direkten Kontakt mit der Oberfläche des für Verderbnis anfälligen Produktes wirken sie auch dort, d.h. sie entfalten ihre Wirkung auf der Oberfläche oder bei Eindringen in das Produkt in diesem selbst.

Der besondere Vorteil des beschriebenen Prozesshilfsmittels ist daher, dass es einerseits zuverlässig dekontaminiert, wobei sich seine Wirksamkeit gegen Grampositive und Gram-negative Bakterien, Pilze, einschließlich Hefen und auch Viren erwiesen hat, während es andererseits für den Konsumenten des Nahrungsmittels keine Gefahr darstellt, da es für diesen vollkommen unschädlich ist und keinerlei mikrobizide, technologische Nachwirkung im Nahrungsmittel besitzt, denn die mikrobizide Wirksamkeit bezieht sich auf das Produktionsumfeld, das durch die erfindungsgemäßen Maßnahmen von kontaminierenden Mikroorganismen befreit wird.

Dabei ist besonders bevorzugt, dass die mikrobiell verderblichen Produkte gleichzeitig durch Zugabe von Additiven und durch eine äußerliche Behandlung mit Prozesshilfsmitteln behandelt werden.

Die in den Prozesshilfsmitteln enthaltenen Aromastoffe sind, wie bereits vorstehend erwähnt, vorzugsweise ausschließlich Naturstoffe, natürliche und naturidentische Aromastoffe, die gemäß FEMA als sicher (GRAS - Generally Reconized As Safe) anerkanrit sind. Die diese FDA-Normen erfüllenden Aromastoffe dürfen "quantum satis" eingesetzt werden, d.h. sie dürfen bis zu der Höchstkonzentration im Nahrungsmittel enthalten sein, in der sie noch keine geruchliche oder geschmackliche Beeinträchtigung des Nahrungsmittels, dem sie zugesetzt werden, bewirken. Die gemäß FEMA aufgeführten Aromastoffe decken sich weitgehend mit den in der entsprechenden europäischen Norm COE enthaltenen Stoffen.

Erfindungsgemäß dürfen außerdem die gemäß Artikel V European Community Directive Flavourings (22.06.88) als "NAT4" klassifizierten Aromastoffe verwendet werden, vorausgesetzt, sie gelten gemäß der zuvor erwähnten FEMA GRAS-Liste als sicher. NAT4-Substanzen sind Substanzen, die unter bestimmten Voraussetzungen als naturidentisch zu deklarieren sind, z.B., wenn diese Substanzen in Verbindung und als Bestandteil mit einem naturlichen oder naturidentischen Aromastoff eingesetzt werden.

Der besondere Vorteil des Prozesshilfsmittels ist, dass es aufgrund seiner in der FEMA GRAS-Liste aufgeführten und von der US-Gesundheitsbehörde FDA, der wohl kritischsten Gesundheitsbehörde überhaupt, als unbedenklich anerkannten Bestandteile im "quantum satis"-Konzentrationsbereich ohne weiteres Nahrungsmitteln zugesetzt werden kann.

Ein weiterer besonderer Vorteil liegt darin, dass die Prozesshilfsmittel den Geschmack und Geruch der behandelten Produkte nicht beeinflussen.

Die erfindungsgemäßen Prozesshilfsmittel werden beispielsweise in Form von Schmiermittetn, Emulgier- und Reinigungsmitteln, Sprühmitteln, Vernebelungsmitteln, gasphasenaktiven Mitteln, Wärmeübertragungsmitteln sowie Schneid- oder Trennmitteln eingesetzt. Ebenso können die Prozesshilfsmittel als Zusätze zu den genannten Mitteln eingesetzt werden. Für weitere Einzelheiten hinsichtlich der Verwendung als Prozesshilfsmittel wird auf die WO 96/29895 verwiesen.

Es ist wesentlich, dass die. Prozesshilfsmittel nicht den mikrobiell verderblichen Produkten (Nahrungsmitteln) beigegeben werden bzw. mit diesen vermischt werden. Vielmehr werden nur die Oberflächen bzw. Schnittflächen der Nahrungsmittel mit den Prozesshilfsmitteln beaufschlagt. Dies kann dadurch geschehen, dass die Nahrungsmitteloberflächen bzw. Schnittflächen direkt mit den Prozesshilfsmitteln beaufschlagt werden. Ebenso ist es aber auch möglich, die Oberflächen von Geräten, Produktionsmaschinen, Verpackungseinrichtungen, Transporteinrichtungen, Verpakkungsmaterialien sowie die Umgebungsluft mit dem Prozesshilfsmittel zu versetzen.

Es ist überraschend, dass die mikrobizide Wirkung der Prozesshilfsmittel bereits bei Anwendung geringer Konzentrationen auftritt. Nur 0,01 bis 5 g/kg, vorzugsweise 0,05 bis 2 g/kg, besonders bevorzugt 0,05 bis 1 g/kg Nahrungsmittel werden bei deren Beaufschlagung verwendet. Bei dem Einsatz für die Umgebungsluft werden nur 0,001 bis 10 $g/m^3$ Luft beispielsweise eingesetzt. Für die Oberflächen von Geräten werden sogar nur 0,000001 bis 0,1 g/$cm^2$ Oberfläche verwendet.

Bei Einhaltung dieser Konzentrationen liegen die in den Nahrungsmitteln nachweisbaren Mengen nur bei 0,001 Gew.-%. Hingegen werden nach dem Stand der Technik regelmäßig 0,1 bis 3 Gew.-% Konservierungsstoff in den Nahrungsmitteln vorhanden sein. Trotz dieser äußerst geringen Konzentrationen ist es erfindungsgemäß überraschend, dass gegenüber herkömmlich konservierten Nahrungsmitteln eine Haltbarkeitsverlängerung von bis zu 50% erzielt werden kann.

Es ist besonders hervorzuheben und erstaunlich, dass bereits durch Prozesshilfsmittel, die indirekt auf Nahrungsmittel aufgebracht werden, bereits 0,001 Gew.-% ausreichen,' um eine Haltbarkeitsstabilisierung bzw. -verbesserung bei erhöhter Produktqualität zu erreichen.

Diese Wirkung ist um so überraschender, als die mikrobizide Wirkungszeit der erfindungsgemäß eingesetzten Aromastoffe unter 24 Stunden, vorzugsweise unter 12 Stunden liegt. Ganz besonders bevorzugt ist es, Prozesshilfsmittel und Konzentrationen so auszuwählen, dass die mikrobizde Wirkungszeit unter 1 Stunde, vorzugsweise unter 15 Minuten liegt.

Im Gegensatz dazu ist es das Ziel der herkömmlichen Konservierungsstoffe, möglichst lange, d.h. über Wochen und Monate, in dem Lebensmittel wirksam zu sein. Trotz der sehr kurzen Wirkungszeiten der erfindungsgemäß eingesetzten Prozesshilfsmittel ist die Haltbarkeit gegenüber den nach dem Stand der Technik mit herkömmlichen Konservierungsstoffen bzw. Konservierungsverfahren behandelten. Lebensmitteln signifikant erhöht. Erfindungsgemäß ist demgemäß bei Kombination der oben beschriebenen Additive und des Prozesshilfsmittels es überraschend Möglich, mit erheblich geringeren Mengen zu arbeiten, als dies beim Einsatz der bisher nach dem Stand der Technik üblichen Konservierungsstoffe erforderlich war.

Im folgenden wird die Erfindung anhand von Beispielen näher beschrieben:

Beispiel 1

Bakteriologische Testverfahren für Additive

- Quantitativer Suspensionstest I (Keimträgerversuch)
- Quantitativer Suspensionstest II (Suspensionsversuch)
- Quantitativer Suspensionstest III (Agardiffusionstest)

Mikroorganismen: Aerobe Mikroorganismen (Gesamtkeimzahl), Enterobacteriaceaen, Enterokokken, Lactobacillen, Hefen, Schimmelpilze. Bei diesen Verfahren können mit unterschiedlichen Mikroorganismen auf unterschiedlichen Nährböden Wirkungen der Additive in Abhängigkeit von der Dosierung und Einwirkzeit ermittelt werden.

Quantitativer Suspensionstest I: - Keimträger-Versuch

| Suspension je nach Testkeim | Ringer Lösung<br>Tryptone Soja Bouillon<br>Chromcult Enterokokken Bouillon<br>Würze-Bouillon | |
| --- | --- | --- |
| Keimträger | 5x5 cm autoklaviertes Baumwolltuch oder Filter | |
| Nähragar | Gesamt-Aerobier < Plate-Count-Agar<br>(Caseinpepton-Glucose-Hefeextrakt Agar)<br>Chromcult | < Enterococcus faecalis<br>Enterococcus faecium<br>Streptococcus bovis |

OGYE-Selektivnährboden (Hefeextrakt-Glucose - Oxytetracyclin)
Mikroorganismen (Schimmelpilze): Aspergillus niger, Saccharomyces

Desoxvcholat - Lactose - Agar

Mikroorganismen:

Lactose-positive - Escherichia coli
Lactose-schwach-positive - Enterobacter (cloacae)
Lactose-schwach-positive - Klebsiella (pneumoniae)
Lactose-negative - Salmonella (typhimurium u. enteritidis), Shigella (flexneri), Proteus (mirabilis), Pseudomonas, Enterococcus (faecalis).

MRS-AGAR (Lactobacillus)

Lactobacillus vulgaris

Baird-Parker-Agar (mit Eigelb-Tellurit-Emulsion)

Mikroorganismen: Staphylococcus aureus, Staphylococcus eqidermidis, Micrococcus (Enterococcus faecium), Bacillus subtilis, Hefen: Endomyces tibuliger.

Cereus-Selektivagar nach Mossel (mit Eigelb-Emulsion)

Mikroorganismen: Bacillus cereus, Bacillus cereus, Bacillus subtilis, Escherichia coli, Pseudomonas aeruginosa, Proteus mirabilis, Staphylococcus aureus.

Desoxycholat-Lactose-Agar

Mikroorganismen:

Lactose-positiv - Escherichia coli
Lactose-schwach-positiv - Enterobacter (chloacae), Klebsiella (pneumaniae).
Lactose-negativ - Salmonella (typhimurium u. enteritidis), Shigella (flexneri), Proteus (mirabilis), Pseudomonas (Enterococcus faecalis).

TGE-Agar (Caseinpepton-Glucose-Fleischextrakt-Agar)

Mikroorganismen: Staphylococcus aureus, Streptococcus agalactiae, Enterococcus faecalis, Escherichia coli, Salmonella typhimurium, Pseudomonas aeruginosa, Bacillus cereus.

Suspensionstest - Quantitativer Keimträgerversuch

| Sonstige | Spezialnährböden und Differenzierungen |
|----------|-----------------------------------------|
| Für | Clostridien, Listerien, u.a. |

Kontamination der Keimträger

Die Kontamination der Keimträger erfolgt nach Einlegen in eine sterile Glasschale durch Übergießen der Testkeimsuspension ($\geq 10^6$/pro ml). Nach 1-10 min langer Lagerung werden die Keimträger in einer mit sterilem Filterpapier ausgelegten Glasschale verteilt und im Brutschrank bei 36°C $\pm$ 1°C getrocknet.

Prüfung

Die kontaminierten und getrockneten Keimträger werden in sterile Glasschalen gelegt und mit je (gr. %/Rezep.) getränkt; 1 h gelagert und für den jeweils vorgesehenen Agar/Testkeim gelegt und im Brutschrank unter der vorgeschriebenen Temperatur bebrütet.

Nach der empfohlenen (Zeit/Bebrütung) werden die Keimträger bei 9-facher Verdünnung (je nach Testkeim) von $10^1$ bis $10^8$ verdünnt und in den jeweils vorgesehenen Agar im Plattengußverfahren eingegeben.

Berechnung:

Alle Agarplatten, die zwischen bis 200 Kolonien aufweisen, werden berücksichtigt. Die Anzahl der Kolonienbildenden Einheiten wird mittels des gewichteten arithmetischen Mittels bestimmt.

# EP 1 096 865 B1

$$\bar{C} = \frac{\Sigma c}{n_1 \times 1 + n_2 \times 0{,}1} \times d$$

$\bar{C}$ =        Anzahl der kolonienbildenden Einheiten je ml/g

$\Sigma c$ =        Summe der Kolonien aller Petrischalen, die zur Berechnung herangezogen werden

$n_1$ =        Anzahl der Petrischalen der niedrigsten Verdünnungsstufe, die zur Berechnung herangezogen werden ($n_1$ = 2 bei 2 Petrischalen)

$n_2$ =        Anzahl der Petrischalen der nächsthöheren Verdünnungsstufe, die zur Berechnung herangezogen werden

$d$ =        Faktor der niedrigsten ausgewerteten Verdünnungsstufe, die auf $n_1$ bezogene Verdünnungsstufe

Quantitativer Suspensionstest II: - Suspensionsversuch

a) Testkeimsuspension mit gewünschtem Testkeim, z.B. $10^6$/ml beimpften 1-60 min einwirken. Gewünschte zu prüfende Rezeptur in vorgesehene Keimsuspensionsröhrchen (unterschiedliche prozentuale Mengen) eingeben. Einwirkzeiten abwarten und in die je nach Keim entsprechenden Agarplatten eingießen oder beimpfen.

b) Testkeimsuspension vor dem Beimpfen der Testkeime (siehe a) mit der gewünschten zu prüfenden Rezeptur behandeln (siehe a). Einwirkzeiten abwarten und dann mit jeweiligen Testkeimen beimpfen und je nach Testkeim die entsprechenden Agarplatten beimpfen, oder eingießen.

Quantitiativer Suspensionstest III: Agar-Diffusionstest

Man gieße Nähragarplatten, die z.B. $10^4$ Mikroorganismen/ml enthalten.
Ein steriles Filterpapierblättchen (10 mm) wird mit der zu prüfenden Rezeptur getränkt und auf die Nähragarplatte gelegt.
Nach der Inkubation von (Zeit/Temperatur je nach Keim) wird die Bildung eines Hemmhofes als positive Reaktion abgelesen.

| Rezepturbeispiele | | | |
|---|---|---|---|
| 23.<br>3 T Benzylalkohol<br>7 T Milchsäure | 24.<br>5 T Zimtsäure<br>5 T Milchsäure<br>5 T Benzylalkohol<br>85 T Sojaöl | Erfindung<br>- Beispiele - | Rezeptur |
| $10^3$<br>$10^3$<br>$10^3$ | $10^3$<br>$10^3$<br>$10^2$ | 5 min. E.Z.<br>15 min. E.Z.<br>60 min. E.Z. | Gesamtkeimzahl |
| | | $10^8$/ml | Kontrolle. |
| $10^3$<br>$10^3$<br>$10^3$ | $10^3$<br>$10^2$<br>$10^2$ | 5 min. E.Z.<br>15 min. E.Z.<br>60 min. E.Z. | Enterobakterien |
| | | $10^8$/ml | Kontrolle |
| $10^3$<br>$10^3$<br>$10^2$ | $10^4$<br>$10^3$<br>$10^3$ | 5 min. E.Z.<br>15 min. E.Z.<br>60 min. E.Z. | Enterokokken |

(fortgesetzt)

| Rezepturbeispiele | | | |
|---|---|---|---|
| 23.<br>3 T Benzylalkohol<br>7 T Milchsäure | 24.<br>5 T Zimtsäure<br>5 T Milchsäure<br>5 T Benzylalkohol<br>85 T Sojaöl | Erfindung<br>- Beispiele - | Rezeptur |
| | | $10^8$/ml | Kontrolle |
| $10^3$<br>$10^2$<br>$10^2$ | $10^2$<br>$10^2$<br>$10^2$ | 5 min. E.Z.<br>15 min. E.Z.<br>60 min. E.Z. | Lactobacille |
| | | $10^5$/ml | Kontrolle |
| $10^3$<br>$10^2$<br>$10^2$ | $10^2$<br>$10^2$<br>$10^2$ | 5 min. E.Z.<br>15 min. E.Z.<br>60 min. E.Z. | Hefen |
| | | $10^5$/ml | Kontrolle |
| $10^2$<br>$10^3$<br>$10^4$ | $10^2$<br>$10^2$<br>$10^2$ | 5 min. E.Z.<br>15 min. E.Z.<br>60 min. E.Z. | Schimmelpilze |
| | | $10^5$/ml | Kontrolle |
| 25.<br>1 T etherisches Öl a)<br>1T etherisches Öl b)<br>300 T Milchsäure<br>698 T Alkohol<br>(Propylenglycol) | 26.<br>97,9T Alkohol<br>(Propylenlglycol)<br>2T Säure (Milchsäure)<br>0,1 T etherisches Öl c) | Erfindung<br>- Beispiele - | Rezeptur |
| $10^2$<br>$10^2$<br>$10^2$ | $10^3$<br>$10^3$<br>103 | 5 min. E.Z.<br>15 min. E.Z.<br>60 min. E.Z. | Gesamtkeimzahl |
| | | $10^8$/ml | Kontrolle |
| $10^2$<br>$10^2$<br>$10^2$ | $10^2$<br>$10^3$<br>$10^3$ | 5 min. E.Z.<br>15 min. E.Z.<br>60 min. E.Z. | Enterobakterien |
| | | $10^8$/ml | Kontrolle |
| $10^2$<br>$10^2$<br>$10^2$ | $10^3$<br>$10^3$<br>$10^3$ | 5 min. E.Z.<br>15 min. E.Z.<br>60 min. E.Z. | Enterokokken |
| | | $10^8$/ml | Kontrolle |
| $10^2$<br>$10^2$<br>$10^2$ | $10^2$<br>$10^2$<br>$10^3$ | 5 min. E.Z.<br>15 min. EZ.<br>60 min. E.Z. | Lactobacille |
| | | $10^5$/ml | Kontrolle |
| $10^2$<br>$10^2$<br>$10^2$ | $10^3$<br>$10^3$<br>$10^3$ | 5 min. E.Z.<br>15 min. E.Z.<br>60 min. E.Z. | Hefen |
| | | $10^5$/ml | Kontrolle |

(fortgesetzt)

| Rezepturbeispiele | | | |
|---|---|---|---|
| 25.<br>1 T etherisches Öl a)<br>1T etherisches Öl b)<br>300 T Milchsäure<br>698 T Alkohol<br>(Propylenglycol) | 26.<br>97,9T Alkohol<br>(Propylenlglycol)<br>2T Säure (Milchsäure)<br>0,1 T etherisches Öl c) | Erfindung<br>- Beispiele - | Rezeptur |
| $10^3$<br>$10^2$<br>$10^2$ | $10^3$<br>$10^3$<br>$10^3$ | 5 min. E.Z.<br>15 min. E.Z.<br>60 min. E.Z. | Schimmelpilze |
| | | $10^5$/ml | Kontrolle |

Beispiel 2

Wirksamkeitstest (Quantitativer Suspensionstest)

Die Wirksamkeit weiterer erfindungsgemäßer Gemische wurde gemäß dem in Beispiel 1 beschriebenen quantitativen Suspensionstest bestimmt. Die Ergebnisse sind in der folgenden Tabelle zusammengefaßt.

| | Gew.-% | Einwirkzeit 1 h | Staph. aureus | Asp. niger |
|---|---|---|---|---|
| | | | Reduktionsfaktor | |
| Propylenglycol<br>Glycerin<br>Anis | 90%<br>9%<br>1% | | 4,9 | 4,0 |
| Propylenglycol<br>Milchsäure<br>Anis | 90%<br>9,9%<br>0,1% | | 6,5 | 4,0 |
| Propylenglycol* | | | 0 | 0 |
| Glycerin* | | | 0 | 0 |
| Wachstumskontolle | | | 7,1 | 5,0 |

\* Vergleichsbeispiel

**Patentansprüche**

**1.** Antimikrobielle Zusammensetzung, die

(A) ein Gemisch ist, das wenigstens zwei GRAS (Generally Recognized As Safe)-Aromastoffe, ausgenommen Polyphenolverbindungen und Benzylalkohol und wenigstens einen hydrophilen, nicht-alkoholischen GRAS-Aromastoff umfasst; oder
(B) ein Gemisch ist, das Benzylalkohol oder Polyphenolverbindungen und wenigstens einen nicht-alkoholischen, hydrophilen GRAS-Aromastoff umfasst, wobei das Gemisch keine weiteren GRAS-Aroma-Alkohole enthält,

wobei der hydrophile, nicht-alkoholische GRAS-Aromastoff eine organische Säure mit 1 bis 15 C-Atomen oder ein physiologisches Salz derselben, ein hydrophiles Acetat oder ein hydrophiler Aldehyd ist und wobei das Gemisch (A) mindestens zwei lipophile GRAS-Aroma-Alkohole, ausgenommen Benzylalkohol umfasst.

**2.** Zusammensetzung nach Anspruch 1, wobei
die organische Säure 2 bis 10 C-Atome aufweist und insbesondere ausgewählt ist aus Essigsäure, Aconitsäure, Ameisensäure, Apfelsäure, Milchsäure, Phenylessigsäure, Citronensäure, Mandelsäure, Weinsäure, Fumarsäure, Tanninsäure, Hydrozimtsäure und deren physiologischen Salzen;

das hydrophile Acetat ausgewählt ist aus Allicin, Triacetin, Kaliumacetat, Natriumacetat und Calciumacetat; und/ oder

der hydrophile Aldehyd ausgewählt ist aus Furfurol, Propionaldehyd und Vanillin.

3. Zusammensetzung nach Anspruch 1 oder 2, wobei die lipophilen GRAS-Aroma-Alkohole (a) ausgewählt sind aus:

n-Butylalkohol, iso-Butylalkohol, Hexylalkohol, L-Menthol, Octylalkohol, Zimtalkohol, α-Methylbenzylalkohol, Heptylalkohol, n-Amylalkohol, iso-Amylalkohol, Anisalkohol, Citronellol, n-Decylalkohol, Geraniol, β-γ-Hexenol, Laurylalkohol, Linalool, Nerolidol, Nonadienol, Nonylalkohol, Rhodinol, Terpineol, Borneol, Clineol, Anisol, Cuminylalkohol, 10-Undecen-1-ol, 1-Hexadecanol oder deren Derivate.

4. Zusammensetzung nach Anspruch 1 bis 3, wobei das Gemisch (A) weiterhin GRAS-Aromastoffe, ausgewählt aus (b) Phenolen, (c) lipophilen Estern, (d) Terpenen, (e) Acetalen, (f) lipophilen Aldehyden, (g) etherischen Ölen, (h) lipophilen Säuren und deren Derivaten, umfasst.

5. Zusammensetzung nach Ansprüchen 1 bis 4, wobei das Gemisch (A) weiterhin einen hydrophilen alkoholischen GRAS-Aromastoff enthält, der ein einwertiger oder mehrwertiger Alkohol mit 2 bis 10, vorzugsweise mit 2 bis 7, C-Atomen ist und der insbesondere ausgewählt ist aus 1-Propanol, Glycerin, Propylenglykol und Acetoin.

6. Zusammensetzung nach Anspruch 1 bis 5, enthaltend 0,01 bis 90 Gew.-%, vorzugsweise 0,1 bis 50 Gew.-% GRAS-Aromastoffe (a) bis (h).

7. Zusammensetzung nach Anspruch 1 oder 2, wobei in dem Gemisch (B) die Polyphenolverbindungen ausgewählt sind aus:

Brenzcatechin, Resorcin, Hydrochinon, Phloroglucin, Pyrogallol, Cyclohexan, Usninsäure, Acylpolyphenolen, Ligninen, Anthocyanen, Flavonen, Catechinen, Gallussäurederivaten, Kaffeesäure, Flavonoiden, Derivaten der genannten Polyphenole und Extrakten aus Camellia Primula.

8. Zusammensetzung nach Anspruch 7, wobei die Polyphenolverbindung Tannin ist.

9. Zusammensetzung nach Anspruch 7 oder 8, enthaltend 0,01 bis 99 Gew.-%, vorzugsweise 0,1 bis 90 Gew.-% Benzylalkohol oder Polyphenolverbindungen und 0,01 bis 50 Gew.-%, vorzugsweise 0,1 bis 30 Gew.-% hydrophile, nicht-alkoholische GRAS-Aromastoffe.

10. Zusammensetzung nach Anspruch 7 bis 9, wobei das Gemisch (B) noch weitere GRAS-Aromastoffe, ausgewählt aus (b) Phenolen, (c) lipophilen Estern, (d) Terpenen, (e) Acetalen, (f) lipophilen Aldehyden, (g) etherischen Ölen, (h) lipophilen Säuren und deren Derivaten enthält.

11. Zusammensetzung nach Anspruch 10, wobei die weiteren GRAS-Aromastoffe Phenole (b) und/oder etherische Öle (g) sind.

12. Zusammensetzung nach Anspruch 10 oder 11, wobei das Gemisch (B) 0,001 bis 25 Gew.-%, vorzugsweise 0,01 bis 9 Gew.-%, der weiteren GRAS-Aromastoffe (b) - (h) enthält.

13. Zusammensetzung nach einem oder mehreren der Ansprüche 1 bis 12, wobei die Zusammensetzung ausschließlich aus GRAS-Aromastoffen besteht.

14. Zusammensetzung nach einem oder mehreren der Ansprüche 1 bis 12, wobei die Zusammensetzung weiterhin Emulgatoren, Stabilisatoren, Antioxidantien, Konservierungsmittel, Lösemittel und/oder Trägerstoffe enthält.

15. Zusammensetzung nach einem oder mehreren der Ansprüche 1 bis 14, wobei die antimikrobielle Zusammensetzung Bestandteil eines Additivs oder eines Prozesshilfsmittels ist.

16. Verfahren zur Haltbarkeitsverbesserung und/oder Stabilisierung von mikrobiell verderblichen Produkten, **dadurch gekennzeichnet, dass** eine antimikrobielle Zusammensetzung, wie in Ansprüchen 1 bis 14 definiert, dem mikrobiell verderblichen Produkt als Additiv zugesetzt wird.

**17.** Verfahren nach Anspruch 16, wobei das Additiv in Mengen von 1 ppm bis 10 Gew.-%, vorzugsweise 0,001 bis 0,5 Gew.-%, und besonders bevorzugt von 0,002 bis 0,25 Gew.-% dem mikrobiell verderblichen Produkt zugesetzt wird.

**18.** Verwendung der antimikrobiellen Zusammensetzung gemäß Ansprüchen 1 bis 14 als Additiv für mikrobiell verderbliche Produkte, insbesondere als Additiv für Lebensmittel und Kosmetika.

**19.** Verfahren zur Haltbarkeitsverbesserung und/oder Stabilisierung von mikrobiell verderblichen Produkten, bei dem vor, nach oder während des Prozesses zur Herstellung, Verarbeitung oder Verpackung der Produkte deren Oberflächen und/oder deren Umgebung, insbesondere die Umgebungsluft und/oder die Oberflächen der unmittelbar mit den Produkten in Kontakt kommenden Geräte oder sonstigen Materialien mit einem oder mehreren Prozesshilfsmitteln beaufschlagt werden, **dadurch gekennzeichnet, dass** das Prozesshilfsmittel eine, wie in Ansprüchen 1 bis 14 definierte, antimikrobielle Zusammensetzung umfasst.

**20.** Verfahren nach Anspruch 19, wobei das Prozesshilfsmittel in Mengen von 0,01 bis 5 g/kg, vorzugsweise 0,05 bis 2 g/kg von Nahrungsmittel, bei Einsatz für die Umgebungsluft in Mengen von 0,001 bis 10 g/m$^3$ Luft, und auf den Oberflächen von Geräten in Mengen von 0,000001 g bis 0,01 g/cm$^2$ eingesetzt wird.

**21.** Verwendung der antimikrobiellen Zusammensetzung gemäß den Ansprüchen 1 bis 14 als Prozesshilfsmittel.

**22.** Mikrobiell verderbliches Produkt enthaltend eine antimikrobielle Zusammensetzung gemäß Ansprüchen 1 bis 14.

**23.** Mikrobiell verderbliches Produkt nach Anspruch 22, ausgewählt aus Lebensmitteln, Kosmetika und Pharmazeutika.

**Claims**

**1.** An antimicrobial composition which is

(A) a mixture comprising at least two GRAS .(generally recognized as safe) flavoring agents, except polyphenol compounds and benzyl alcohol, and at least one hydrophilic non-alcoholic GRAS flavoring agent; or

(B) a mixture comprising benzyl alcohol or polyphenol compounds and at least one non-alcoholic hydrophilic GRAS flavoring agent, the mixture containing no other GRAS flavor alcohols;

wherein said hydrophilic non-alcoholic GRAS flavoring agent is an organic acid containing from 1 to 15 carbon atoms or its physiologically acceptable salt, a hydrophilic acetate or a hydrophilic aldehyde, and wherein mixture (A) comprises at least two lipophilic GRAS flavor alcohols, except benzyl alcohol.

**2.** The composition according to claim 1, wherein said organic acid contains from 2 to 10 carbon atoms and is selected, in particular, from acetic acid, aconitic acid, formic acid, malic acid, lactic acid, phenylacetic acid, citric acid, mandelic acid, tartaric acid, fumaric acid, tannic acid, hydrocinnamic acid and their physiologically acceptable salts; said hydrophilic acetate is selected from allicin, triacetin, potassium acetate, sodium acetate and calcium acetate; and/or said hydrophilic aldehyde is selected from furfurol, propionic aldehyde and vanillin.

**3.** The composition according to claim 1 or 2, wherein said lipophilic GRAS flavor alcohols (a) are selected from n-butyl alcohol, iso-butyl alcohol, hexyl alcohol, L-menthol, octyl alcohol, cinnamyl alcohol, $\alpha$-methylbenzyl alcohol, heptyl alcohol, n-amyl alcohol, iso-amyl alcohol, anisic alcohol, citronellol, n-decyl alcohol, geraniol, $\beta$-$\gamma$-hexenol, lauryl alcohol, linalool, nerolidol, nonadienol, nonyl alcohol, rhodinol, terpineol, borneol, clineol, anisole, cuminyl alcohol, 10-undecen-1-ol, 1-hexadecanol, or their derivatives.

**4.** The composition according to claims 1 to 3, wherein mixture (A) additionally contains GRAS flavoring agents selected from (b) phenols, (c) lipophilic esters, (d) terpenes, (e) acetals, (f) lipophilic aldehydes, (g) essential oils, (h) lipophilic acids, and their derivatives.

**5.** The composition according to claims 1 to 4, wherein mixture (A) additionally contains a hydrophilic alcoholic GRAS flavoring agent which is a monohydric or polyhydric alcohol containing from 2 to 10 carbon atoms, preferably from 2 to 7 carbon atoms, and is selected, in particular, from 1-propanol, glycerol, propylene glycol and acetoin.

6.  The composition according to claims 1 to 5, containing from 0.01 to 90% by weight, preferably from 0.1 to 50% by weight, of GRAS flavoring agents (a) to (h).

7.  The composition according to claim 1 or 2, wherein the polyphenol compounds in mixture (B) are selected from pyrocatechol, resorcinol, hydroquinone; phloroglucinol, pyrogallol, hexahydroxybenzene, usnic acid, acylpolyphenols, lignins, anthocyans, flavones, catechols, gallic acid derivatives, caffeic acid, flavonoids, derivatives of the mentioned polyphenols, and extracts from Camellia, Primula.

8.  The composition according to claim 7, wherein said polyphenol compound is tannin.

9.  The composition according to claim 7 or 8, containing from 0.01 to 99% by weight, preferably from 0.1 to 90% by weight, of benzyl alcohol or polyphenol compounds, and from 0.01 to 50% by weight, preferably from 0.1 to 30% by weight, of hydrophilic non-alcoholic GRAS flavoring agents.

10. The composition according to claims 7 to 9, wherein said mixture (B) contains additional GRAS flavoring agents selected from (b) phenols, (c) lipophilic esters, (d) terpenes, (e) acetals, (f) lipophilic aldehydes, (g) essential oils, (h) lipophilic acids, and their derivatives.

11. The composition according to claim 10, wherein said additional GRAS flavoring agents are phenols (b) and/or essential oils (g).

12. The composition according to claim 10 or 11, wherein said mixture (B) contains from 0.001 to 25% by weight, preferably from 0.01 to 9% by weight, of said additional GRAS flavoring agents (b) to (h).

13. The composition according to one or more of claims 1 to 12, wherein said composition exclusively consists of GRAS flavoring agents.

14. The composition according to one or more of claims 1 to 12, wherein said composition additionally contains emulsifiers, stabilizers, antioxidants, preservatives, solvents and/or carriers.

15. The composition according to one or more of claims 1 to 14, wherein said antimicrobial composition is part of an additive or of a processing aid.

16. A method for the improvement and/or stabilization of the keeping quality of microbially perishable products, **characterized in that** an antimicrobial composition as defined in claims 1 to 14 is added as an additive to said microbially perishable product.

17. The method according to claim 16, wherein said additive is added to said microbially perishable product in amounts of from 1 ppm to 10% by weight, preferably from 0.001 to 0.5% by weight, more preferably from 0.002 to 0.25% by weight.

18. Use of the antimicrobial composition according to claims 1 to 14 as an additive for microbially perishable products, especially as an additive for food products and cosmetics.

19. A method for the improvement and/or stabilization of the keeping quality of microbially perishable products in which the surfaces of the products and/or their environment, especially the ambient air and/or the surfaces of the equipment or other materials immediately contacting the products, are treated with one or more processing aids before, after or during the process for the manufacturing, processing or packaging of the products, **characterized in that** said processing aid comprises an antimicrobial composition as defined in claims 1 to 14.

20. The method according to claim 19, wherein said processing aid is employed in amounts of from 0.01 to 5 g/kg, preferably from 0.05 to 2 g/kg, for food products, in amounts of from 0.001 to 10 $g/cm^2$ of air when used for the ambient air, and in amounts of from 0.000001 g to 0.1 $g/cm^2$ on the surfaces of equipment.

21. Use of the antimicrobial composition according to claims 1 to 14 as a processing aid.

**22.** A microbially perishable product containing the antimicrobial composition according to claims 1 to 14.

**23.** The microbially perishable product according to claim 22, which is selected from food products, cosmetics and pharmaceuticals.

**Revendications**

**1.** Composition antimicrobienne qui est :

(A) un mélange comprenant au moins deux arômes GRAS (*Generally Recognized As Safe*), à l'exception des composés polyphénoliques et de l'alcool benzylique, et au moins un arôme GRAS hydrophile non alcoolique ; ou
(B) un mélange comprenant de l'alcool benzylique ou des composés polyphénoliques et au moins un arôme GRAS hydrophile non alcoolique, le mélange ne contenant aucun autre arôme alcoolique GRAS,

dans laquelle l'arôme GRAS hydrophile non alcoolique est un acide organique ayant de 1 à 15 atomes de carbone ou un de ses sels physiologiques, un acétate hydrophile ou un aldéhyde hydrophile, et dans laquelle le mélange (A) comprend au moins deux arômes alcooliques lipophiles GRAS, à l'exception de l'alcool benzylique.

**2.** Composition selon la revendication 1, dans laquelle

- l'acide organique présente de 2 à 10 atomes de carbone et, en particulier, est choisi parmi l'acide acétique, l'acide aconitique, l'acide formique, l'acide malique, l'acide lactique, l'acide phénylacétique, l'acide citrique, l'acide mandélique, l'acide tartrique, l'acide fumarique, l'acide tannique, l'acide hydrocinnamique, ainsi que des sels physiologiques de ceux-ci ;
- l'acétate hydrophile est choisi parmi l'allicine, la triacétine, l'acétate de potassium, l'acétate de sodium et l'acétate de calcium ; et/ou
- l'aldéhyde hydrophile est choisi parmi le furfurol, le propionaldéhyde et la vanilline.

**3.** Composition selon la revendication 1 ou la revendication 2, dans laquelle les arômes alcooliques lipophiles GRAS (a) sont choisis parmi :

l'alcool n-butylique, l'alcool isobutylique, l'alcool hexylique, le L-menthol, l'alcool octylique, l'alcool cinnamique, l'alcool α-méthylbenzylique, l'alcool heptylique, l'alcool n-amylique, l'alcool isoamylique, l'alcool anisique, le citronellol, l'alcool n-décylique, le géraniol, le β-γ-hexénol, l'alcool laurylique, le linalol, le nérolidol, le nonadiénol, l'alcool nonylique, le rhodinol, le terpinéol, le bornéol, le clinéol, l'anisol, l'alcool cuminylique, le 10-undécén-1-ol, le 1-hexadécanol, ou des dérivés de ceux-ci.

**4.** Composition selon les revendications 1 à 3, dans laquelle le mélange (A) comprend en plus des arômes GRAS choisis parmi (b) des phénols, (c) des esters lipophiles, (d) des terpènes, (e) des acétals, (f) des aldéhydes lipophiles, (g) des huiles essentielles, (h) des acides lipophiles, ainsi que des dérivés de ceux-ci.

**5.** Composition selon les revendications 1 à 4, dans laquelle le mélange (A) contient en plus un arôme alcoolique hydrophile GRAS qui est un alcool univalent ou multivalent ayant de 2 à 10, de préférence de 2 à 7 atomes de carbone et qui est choisi en particulier parmi le 1-propanol, le glycérol, le propylène glycol et l'acétine.

**6.** Composition selon les revendications 1 à 5, contenant de 0,01 à 90% en poids, de préférence de 0,1 à 50% en poids des arômes (a) à (h).

**7.** Composition selon la revendication 1 ou la revendication 2, dans laquelle, dans le mélange (B), les composés polyphénoliques sont choisis parmi :

la brenzcatéchine, la résorcine, l'hydroquinone, la phloroglucine, le pyrogallol, le cyclohexane, l'acide usnique, des acylpolyphénols, des lignines, des anthocyanes, des flavones, des catéchines, des dérivés de l'acide gallique, l'acide caféique, des flavonoïdes, des dérivés des polyphénols précités et des extraits de *Camellia primula*.

**8.** Composition selon la revendication 7, dans laquelle le composé polyphénolique est la tannine.

**9.** Composition selon la revendication 7 ou la revendication 8, contenant de 0,01 à 99% en poids, de préférence de 0,1 à 90% en poids d'alcool benzylique ou de composés polyphénoliques et de 0,01 à 50% en poids, de préférence de 0,1 à 30% en poids d'arômes hydrophiles non alcooliques GRAS.

**10.** Composition selon les revendications 7 à 9, dans laquelle le mélange (B) contient en plus d'autres arômes GRAS choisis parmi (b) des phénols, (c) des esters lipophiles, (d) des terpènes, (e) des acétals, (f) des aldéhydes lipophiles, (g) des huiles essentielles, (h) des acides lipophiles, ainsi que des dérivés de ceux-ci.

**11.** Composition selon la revendication 10, dans laquelle les autres arômes GRAS sont des phénols (b) et/ou des huiles essentielles (g).

**12.** Composition selon la revendication 10 ou la revendication 11, dans laquelle le mélange (B) contient de 0,001 à 25% en poids, de préférence de 0,01 à 9% en poids des autres arômes GRAS (b) à (h).

**13.** Composition selon l'une ou plusieurs des revendications 1 à 12, dans laquelle la composition est constituée exclusivement par des arômes GRAS.

**14.** Composition selon l'une ou plusieurs des revendications 1 à 12, dans laquelle la composition contient en plus des émulsifiants, des stabilisants, des antioxydants, des conservateurs, des solvants et/ou des véhicules.

**15.** Composition selon l'une ou plusieurs des revendications 1 à 14, dans laquelle la composition antimicrobienne est un constituant d'un additif ou d'un auxiliaire de procédé.

**16.** Procédé d'amélioration de la durée de conservation et/ou de stabilisation de produits périssables sous l'action de microbes, **caractérisé en ce que** l'on ajoute au produit périssable sous l'action de microbes une composition antimicrobienne telle que définie dans les revendications 1 à 14 en tant qu'additif.

**17.** Procédé selon la revendication 16, dans lequel l'additif est ajouté au produit périssable sous l'action de microbes en des quantités allant de 1 ppm à 10% en poids, de préférence de 0,001 à 0,5% en poids et de façon particulièrement préférée de 0,002 à 0,25% en poids.

**18.** Utilisation de la composition antimicrobienne selon les revendications 1 à 14 comme additif pour des produits périssables sous l'action de microbes, en particulier comme additif pour des produits alimentaires et des cosmétiques.

**19.** Procédé d'amélioration de la durée de conservation et/ou de stabilisation de produits périssables sous l'action de microbes, dans lequel, avant, pendant ou après le processus de fabrication, de transformation ou de conditionnement des produits, on applique sur leurs surfaces et/ou dans leur environnement, en particulier dans l'air ambiant et/ou sur les surfaces des appareils ou autres matériaux venant directement en contact avec les produits, un ou plusieurs auxiliaires de procédé, **caractérisé en ce que** l'auxiliaire de procédé comprend une composition antimicrobienne telle que définie dans les revendications 1 à 14.

**20.** Procédé selon la revendication 19, dans lequel l'auxiliaire de procédé est utilisé en des quantités allant de 0,01 à 5 g/kg, de préférence de 0,05 à 2 g/kg de produit alimentaire, en des quantités allant de 0,001 à 10 g/m$^3$ d'air dans le cas de l'application dans l'air ambiant et en des quantités allant de 0,000001 g à 0,01 g/cm$^2$ dans le cas de l'application sur des surfaces d'appareils.

**21.** Utilisation de la composition antimicrobienne selon les revendications 1 à 14 comme auxiliaire de procédé.

**22.** Produit périssable sous l'action de microbes, contenant une composition antimicrobienne selon les revendications 1 à 14.

**23.** Produit périssable sous l'action de microbes selon la revendication 22, choisi parmi des produits alimentaires, des cosmétiques et des produits pharmaceutiques.